# EUROPÄISCHE PATENTSCHRIFT

(11) **EP 3 285 685 B1**
(45) Veröffentlichungstag und Bekanntmachung des Hinweises auf die Patenterteilung: **21.08.2024**
(21) Anmeldenummer: 16723025.9
(22) Anmeldetag: 20.04.2016
(51) Int. Cl.: A61F 2/07, A61F 2/915

(54) **STENT-GRAFT**
STENT GRAFT
ENDOPROTHÈSE COUVERTE

(30) Priorität: 21.04.2015 DE 102015106052
(43) Veröffentlichungstag der Anmeldung: 28.02.2018
(73) Patentinhaber: Bentley InnoMed GmbH, 72379 Hechingen (DE)
(72) Erfinder: OBRADOVIC, Nikola, 79639 Lorrach (DE)
(74) Vertreter: Hoefer & Partner Patentanwälte mbB
(86) Internationale Anmeldenummer: PCT/EP2016/058694
(87) Internationale Veröffentlichungsnummer: WO 2016/169949

(56) Entgegenhaltungen:
- WO-A1-2005/087138
- DE-A1- 102012 001 996

## Beschreibung

Die Erfindung betrifft einen Stent-Graft aus einem Stent mit einer Mehrzahl von nebeneinander angeordneten und durch Verbindungstege miteinander verbundenen Ringsegmenten und wenigstens einer Membran. Ferner betrifft die Erfindung die Verwendung eines solchen Stent-Grafts zur Behandlung vaskulärer Fehlbildungen.

Stent-Grafts dieser Art werden in Blutgefäßen eingesetzt, etwa um anomal verengte, erweiterte oder auch geschädigte Blutgefäße zu stützen. Die Kombination von Stent und Membran dient dabei der Versorgung auch ausgedehnterer, behandlungsbedürftiger Gefäßabschnitte, welche eine größere Länge und vor allem auch Flexibilität des Implantats voraussetzen. Insbesondere werden Stent-Grafts bei der Überbrückung von Gefäßfehlbildungen eingesetzt, etwa um Aneurysmen von der Blutzirkulation auszuschließen. Zur Implantation solcher Stent-Grafts werden in der Regel Ballonkatheter verwandt.

Im Stand der Technik ist es bekannt, für diese Aufgabe Stent-Grafts einzusetzen, welche aus zwei Stents und einer flexiblen Membran, beispielsweise aus Teflon bestehen. Einen solchen Stent-Graft beschreibt die Druckschrift EP 2 151 217 A1. Der offenbarte Stent-Graft besteht aus einem inneren Stent und einem koaxial um diesen ersten angeordneten äußeren Stent, zwischen welchen eine flexible, dehnbare Membran angeordnet ist. Die Endbereiche der Stents mit der dazwischen angeordneten Membran werden verschweißt.

Die EP 1 266 635 A2 offenbart einen Stent-Graft, welcher einen zylindrischen Stent und eine zylindrische Membran aufweist, die beispielsweise über Nähte oder Haken miteinander verbunden sind. Zusätzlich oder alternativ kann die Verbindung durch ein geringes Überlappen von Stent und Membran gesichert werden.

Die WO 2009/035679 A1 offenbart einen Stent-Graft, welcher einen durchgehenden inneren Liner aus Polyester oder ePTFE aufweist. An einem Bereich dieses inneren Liners ist ein Stent angeordnet, welcher den inneren Liner koaxial umschließt. Die Bereiche des inneren Liners neben dem Stent sind mit einer zweiten Schicht aus Polyester oder ePTFE ummantelt, um die Wandstärke des Implantats in dem nicht durch den Stent gestützten Bereich zu erhöhen. Die Endbereiche von Stent und zweiter Schicht werden aneinander geschoben. Wenn erforderlich kann zusätzlich Verstärkungsmaterial, z. B. ePTFE, auf die Außenseite der Stent-Graft-Komponenten, insbesondere an den Übergängen zwischen Stent und zweiter Schicht, aufgebracht werden.

Von den genannten Stent-Grafts mit einer Verbindung von Membran und einem Stent verbindet lediglich der in der EP 1 266 635 A2 gezeigte genau einen Stent mit genau einer Membran. Die anderen Lösungen im Stand der Technik erfordern stets einen weiteren Stent oder eine weitere Membran, welche zur Verstärkung koaxial um den ersten Stent bzw. die erste Membran angeordnet sind.

Die Lösung gemäß der EP 1 266 635 A2 verwendet für die Verbindung zwischen Stent und Membran Haken oder Fäden und/oder alternativ ein Überlappen beider Bestandteile. Problematisch ist dabei stets die Haltbarkeit der Haken und Fäden, welche großen Reibungskräften innerhalb des Blutgefäßes ausgesetzt sind. Ebenso besteht die Gefahr der Gewebereizung oder -verletzung durch abstehende Haken oder überstehende Kanten zwischen Stent und Membran.

Stent-Grafts sind ferner in der WO 2012/084202 A1 und der WO 01/66035 A2 beschrieben. Hier wird die Membran an den randständigen Ringsegmente festgelegt, was zur Folge hat, dass die Expansion des Stents bei der Platzierung die Fixierung der Membran am Stent beeinträchtigen kann.

DE102012001996 A1 zeigt ein Stent-Graft aus einem Stent mit einer Mehrzahl von nebeneinander angeordneten und miteinander verbundenen Ringsegmenten, wenigstens einer Membran, und wenigstens einem randständigen Ringsegment mit mäandrierendem Stegverlauf, bei dem einwärts- oder auswärts weisende Stegschlingen eingeschnitten sind, dergestalt, dass Federzungen entstehen, die formschlüssig in den Stegschlingen angeordnet sind und gegen den Stegverlauf federnd beweglich sind, wobei die Membran zwischen Federzungen und Stegschlingen eingeklemmt ist und wenigstens eine Membran als Lochmembran ausgebildet ist.

Es ist Aufgabe der vorliegenden Erfindung, einen Stent-Graft zu schaffen, welcher Stent und Membran einfach und haltbar miteinander verbindet, wenig Gefäßlumen verbraucht und keine Gewebereizung oder -verletzung verursacht. Die Verbindung zwischen Membran und Stent soll insbesondere nicht an den Ringsegmenten erfolgen, um eine sichere Fixierung der Membran zu ermöglichen.

Diese Aufgabe löst die Erfindung mit dem Stent-Graft der eingangs bezeichneten Art, bei der eine Mehzahl von Verbindungsstegen zwischen benachbarten Ringsegmenten Federzungen aufweisen, die formschlüssig in den Verbindungsstegen angeordnet sind und gegen die Verbindungsstege federnd beweglich sind, wobei die Membran zwischen Federzungen und Verbindungsstegen eingeklemmt ist.

Der erfindungsgemäße Stent-Graft besteht aus einer Mehrzahl von miteinander verbundenen mäandrierenden Ringsegmenten. Diese Ringsegmente entsprechen den Ringsegmenten herkömmlicher Stents, wie sie vielfach vorgeschlagen und in Gebrauch sind. Erfindungsgemäß ausgestaltet und verändert sind Verbindungsstege zwischen zwei Ringsegmenten in die Federzungen eingeschnitten sind. Die Verbindungsstege mit Federzungen verbinden insbesondere randständige Ringsegmente mit benachbarten Ringsegmenten.

Unter randständigen Ringsegmenten werden solche Ringsegmente verstanden, die am Ende des Stents angeordnet sind, also den Stent an seinen Enden begrenzen. Dabei kann erfindungsgemäß nur eines dieser randständigen Ringsegmente über Verbindungsstege mit Federzungen mit dem benachbarten Ringsegment verbunden sein, vorzugsweise sind es allerdings die randständigen Ringsegmente an beiden Stentenden.

Unter mäandrierendem Stegverlauf der randständigen Ringsegmente wird sowohl ein wellenförmiger Stegverlauf als auch ein zickzackförmiger Stegverlauf verstanden. Wellenförmige und zickzackförmige Stegverläufe und Ringsegmente sind bei vaskulären Stents weitgehend üblich, um die bei der Expansion auftretende Längenkontraktion zumindest teilweise auszugleichen.

Zur Ausbildung der Federzungen werden die Verbindungsstege partiell eingeschnitten, so dass sich gegen den Stegverlauf bewegliche Federzungen ausbilden. Dabei können entweder einwärts- oder auswärtsweisenden Federungen eingeschnitten sein. Die Federzungen selbst sind im Fertigungszustand des Stents (also vor dem Aufkrimpen auf einen Ballon bzw. vor der Expansion) formschlüssig in die Stege eingepasst.

Um durch die Einschnitte in die Verbindungsstege die Stegfestigkeit nicht zu verschlechtern, ist es angebracht, die Stegbreite im Bereich der Ausbildung der Federzungen zu erhöhen, etwa zu verdoppeln oder zu verdreifachen. Dies bedeutet, dass die Federzungen eine im Wesentlichen normale Stegbreite aufweisen können wie auch die seitlich an die Federzungen angrenzenden Stegelemente der Verbindungsstege.

Die Befestigung der Membran mit Hilfe von nebeneinander an dem Stent angeordneten Federzungen ermöglicht eine einfache und sichere Verbindung von Stent und Membran. Die in den Stegschlingen angeordneten Federzungen werden beispielsweise durch Laserschneiden erzeugt. Das Einklemmen der Membran unter die Federzungen ist fertigungstechnisch leicht zu bewerkstelligen. Die Integration der Verbindungselemente zwischen Stent und Membran als Federzungen in den Stent beseitigt dabei die Gefahr, das sich Verbindungsbestandteile vom Stent lösen und frei beweglich in die Blutbahn gelangen, wie dies bei anderen Verbindungselementen vorkommen kann. Gleichzeitig vermeidet diese Art der Integration eine Ausweichung der Stentexpansion auf die Membranfixierung.

Zur Erzeugung einer gleichmäßigen Verbindung zwischen Stent und Membran ist es sinnvoll, eine größere Anzahl von nebeneinander in die gleiche Richtung weisenden Federzungen anzuordnen. Insbesondere sind alle in die gleiche Richtung weisenden Verbindungsstege des jeweiligen randständigen Ringsegments mit einer Federzunge ausgestattet. Diese Federzungen sind dann parallel zur Längsachse des Stents ausgerichtet. Sie weisen vorzugsweise stentauswärts. Die Form der Federzungen entspricht dabei weitgehend der Form der Verbindungsstege.

In der Regel sind die Federzungen parallel zur Längsachse des Stents ausgerichtet. Bei entsprechender Gestaltung der Einschnitte und Anlage der Verbindungsstege sind aber auch Abweichungen von der Parallelität möglich.

Die Anzahl der Federzungen zwischen zwei randständigen Ringsegmenten richtet sich grundsätzlich nach der gewünschten Festigkeit der jeweiligen Verbindung zwischen Stent und Membran. In der Regel werden mindestens zwei gegenüberliegende Verbindungsstege eines randständigen Ringsegments mit Federzungen ausgestattet sein, vorzugsweise alle Verbindungsstege des randständigen Ringsegments zum benachbarten Ringsegment.

Der erfindungsgemäße Stent-Graft wird in der Regel einen mittels eines Ballonkatheters aufweitbaren Stent, hergestellt beispielsweise aus einem medizinisch akzeptablen Stahl, aufweisen. Alternativ sind auch solche Varianten denkbar, bei welchen der Stent selbstexpandierend ausgebildet ist, z. B. durch Verwendung einer Formgedächtnislegierung wie Nitinol.

Die Membran, in der Regel eine Folie oder ein Schlauch, kann aus allen in der Medizintechnik üblichen und zugelassenen Materialien bestehen. Insbesondere eignen sich aber PTFE und Polyester. Besonders bevorzugt ist eine Membran aus ePTFE. Die Membran kann dabei zusätzlich funktionell beschichtet sein, z. B. mit entzündungshemmenden, proliferationshemmenden oder therapeutischen Stoffen, beispielsweise mit Rapamycin, Paclitaxel oder Heparin. Die Membran ist vorzugsweise schlauchförmig ausgebildet und weist das notwendige Dehnungsvermögen auf, um bei der Stentexpansion mitzugehen.

Die Federzungen der Verbindungsstege der randständigen Ringsegmente mit mäandrierendem Stegverlauf können stenteinwärts oder -auswärts ausgerichtet sein und weisen vorzugsweise stentauswärts. Die in den Federzungen eingeklemmte Membran kann zur Verbesserung des Sitzes im Endbereich, der unter die Federzungen zu liegen kommt, auf sich selbst zurückgefaltet sein, um einen besseren Klemmeffekt zu erzielen.

Der erfindungsgemäße Stent-Graft kann die Membran innen oder außen aufweisen. Bevorzugt ist eine Anordnung der Membran an der Außenseite. Diese Anordnung hat den Vorteil, dass die Einwirkung der Stentstruktur auf die Gefäßwand durch die zwischenliegende Membran gemildert wird. Insbesondere ist die Membran an beiden Enden des Stents in den randständigen Ringsegmenten festgeklemmt.

Möglich ist ferner eine Variante, bei der die Membran innenliegend im Stent in den Federzungen eingeklemmt ist, außen über die Stentoberfläche läuft und am entgegengesetzten Ende wiederum innenliegend von den Federzungen gehalten wird. In diesem Fall weisen die Federzungen vorzugsweise stentauswärts. Gleichermaßen kann die Membran innenliegend am Stent angeordnet sein und auf der Außenseite festgeklemmt sein. Eine Kombination aus Außen- und Innenklemmung ist ebenfalls möglich.

Vorzugsweise ist die Membran zusätzlich durch Verkleben an randständigen Ringsegmenten festgelegt. Dies kann durch einen körperverträglichen Klebstoff erfolgen, vorzugsweise aber über ein Klebefand, das in der Höhe der Federzungen über die Membran geklebt wird. Ein solches Klebeband dient zusätzlich zu dem Sicherungsaspekt auch dazu, die Gefäßwand vor dem direkten Kontakt mit den Federzungen des randständigen Ringsegments zu schützen.

Die Stent-Grafts gemäß der Erfindung können die Membran an beliebiger Stelle und auch mehr als eine Membran aufweisen. Beispielsweise kann die Membran nur an einem oder am anderen Ende des Stents oder mittig im Stent angeordnet sein und Stent-Bereiche frei lassen. Des Weiteren ist es möglich, mehr als nur eine Membran vorzusehen, die beispielsweise mit einem frei bleibenden Zwischenraum an den Enden angeordnet sind. Wenn mehr als nur eine Membran vorhanden ist, ist jede für sich über entsprechende Federzungen und ggf. durch Verkleben am Stentgerüst festgelegt.

Sind beispielsweise zwei Membranen endständig angeordnet, kann der dazwischenliegende nicht von einer Membran abgedeckte Teil des Stent-Grafts im Bereich einer Gefäßabzweigung angeordnet werden, so dass der Blutfluss in das abzweigende Gefäß nicht beeinträchtigt wird. Zu diesem Zweck können auch die beiden Stenthälften, die die Membran aufweisen, durch sogenannten Verbinder nur lose miteinander verbunden sein. Dies bringt zudem eine erhöhte Flexibilität in diesen Bereich mit sich.

Zur Erhöhung der Flexibilität können Stents, insbesondere auch die erfindungsgemäßen Stent-Grafts, auch Einkerbungen aufweisen. Diese Einkerbungen befinden sich vorzugsweise auf Verbindungsstegen zwischen zwei Ringsegmenten. Verbindungsstege mit Einkerbungen weisen zweckmäßigerweise keine Federzungen auf. Die Einkerbungen erlauben insbesondere eine Anpassung des Stent-Grafts an einen gekrümmten Gefäßverlauf und eine trompetenförmige Aufweitung des Stentendes bei sogenannten Fenestrierungen. Bei letzteren handelt es sich um in Abzweigungen angeordnete Stents, die von einem ebenfalls mit einem Stent ausgestatteten Hauptgefäß ausgehen und an ein "Fenster" des Stents im Hauptgefäß ausgeschlossen sind. Benötigt werden solche Fenestrierungen beispielsweise bei Aortenaneurysmen, die mit einem Stent überbrückt werden und bei denen ein abgehendes Gefäß frei gehalten und ebenfalls mit einem Stent ausgestattet werden muß.

Die Einkerbungen befinden sich zweckmäßigerweise auf alten Verbindungsstegen zwischen den jeweiligen Ringsegmenten. Ein Stent-Graft kann auch im Verlauf mehrfach mit Einkerbungen versehen sein, d. h. zwischen mehr als nur zwei Ringsegmenten. Die Einkerbungen können "echte" Kerben mit einem Öffnungswinkel von beispielsweise 60 bis 120 ° sein, die gerade verlaufende Schenkel aufweisen, aber auch gerundete Eintiefungen, etwa halbkreisförmig. Die Einkerbung sollte eine Tiefe von nicht mehr als etwa 1/3 der Stegdicke aufweisen, insbesondere nicht mehr als 1/4. Die Stegbreite kann im Bereich der Einkerbung ebenfalls reduziert sein, beispielsweise um 1/4 gegenüber der normalen Stegbreite. Die Eintiefung ist in jedem Fall deutlich geringer als die Stegbreite und beträgt maximal 1/4 der Stegbreite.

Die erfindungsgemäßen Stent-Grafts werden in erster Linie zur Behandlung vaskulärer Fehlbildungen eingesetzt. Dabei kann es sich um das Abschließen von abzweigenden Gefäßen handeln, aber auch um das Stilllegen von Aneurysmen oder arteriovenösen Shunts.

Möglich sind ferner Ausführungsformen, bei denen der erfindungsgemäße Stent-Graft zwei Stenteinheiten aufweist, die dazu dienen, eine dazwischenliegende Schlauchmembran in ein Gefäß einzuspannen. In diesem Fall weist der Stent die Klemmverbindung zur Festlegung der Membran nur an einem Ende auf; das andere Ende der Schlauchmembran ist mit dem zweiten Stent verbunden. Ein solcher Stent-Graft kann in großflächig geschädigte Gefäße implantiert werden, beispielsweise nach Obliteration der Epithelzellschicht eines Blutgefäßes.

Gemäß einer besonders bevorzugten Ausführungsform können die Federzungen in den Verbindungsstegen im Endbereich oder in ihrem Verlauf eine Abwinkelung oder Verzweigung aufweisen, dergestalt, dass sie beispielsweise einen rechten Winkel beschreiben. Damit entsteht zwischen der Federzunge und der eingeklemmten Membran eine größere Kontaktfläche, die die Fixierwirkung der Federzungen verbessert. Auch in diesem Fall bleibt die formflüssige Einpassung der Federzungen in den Verbindungsstegen bei freier Beweglichkeit gegen den Verlauf der Verbindungsstege bestehen. Im Bereich der Abwicklung weisen die Verbindungsstege entsprechend einen Bogen auf, der um den abgewinkelten Endbereich der Federzunge herumführt.

Gemäß einer weiteren bevorzugten Ausführungsform kann wenigstens eines des beiden Ringsegmente, zwischen denen die Verbindungsstege mit den Federzungen verlaufen, frei endende Stege aufweisen, die parallel zu den Federzungen ausgerichtet sind. Diese Stege sind an den Spitzen oder Wendepunkten der Ringsegmente angeordnet und haben ebenfalls, wegen des freien Endes eine Federwirkung. Die Membran kann dann entsprechend alternierend oberhalb und unterhalb der Federzungen in den Verbindungsstegen und den frei endenden Stegen angeordnet werden. Auch dies erhöht die Kontaktfläche zu der eigespannten Membran und dient damit der Sicherung der Membran am Stent.

Die Erfindung wird im Folgenden anhand der Abbildungen näher erläutert. Es zeigen:
- Fig. 1: einen ersten erfindungsgemäß modifizierten Stent,
- Fig. 2: einen zweiten erfindungsgemäß modifizierten Stent, und
- Fig. 3: den Stent gemäß Fig.2 mit eingespannter Membran, und
- Fig. 4: einem Stent mit Einkerbungen

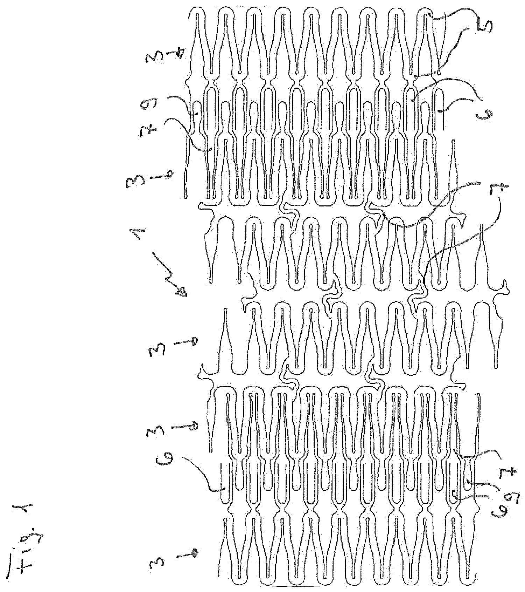

Fig. 1 zeigt einen erfindungsgemäß modifizierten Stent 1, wie er für die Festlegung einer Membran über Federzungen 6 modifiziert ist. Der Stent 1 besteht aus einer Mehrzahl von Ringsegmenten 3, die einen im Wesentlichen zickzackförmigen Verlauf haben. In der Abbildung ist der Stent 1 in aufgeschnittenem und ausgebreitetem Zustand gezeigt; im Originalzustand nach der Fertigung ist er ein aus Stegen zusammengesetztes und mit Durchbrechungen versehenes Rohr, beispielsweise aus einem medizinischem Stahl oder Nitinol. Der Fertigung erfolgt auf an und für sich bekannte Weise durch Laserschneiden eines geeignet dimensionierten Rohres.

Die Ringsegmente 3 sind durch Dehnungselemente 7 miteinander verbunden dergestalt, dass bei Platzierung des Stents über einen Ballon die Längenreduktion, die sich aus der Expansion der Ringsegmente 3 ergibt, zumindest teilweise durch eine Streckung der Verbindungselemente 7 kompensiert wird.

Das randständige Ringsegment 3 am Stentende hat einen mäandrierenden Verlauf. Ein zickzackförmiger Verlauf wäre ebenfalls möglich. Die einzelnen Stege bilden Stegschlingen 5 aus, die wellen- oder mäanderförmig über die Zirkumferenz des Stents verlaufen.

Die Verbindungsstege 7, die von den endständigem Ringsegmenten 3 ausgehen, weisen Einschnitte auf, die Federzungen 6 entstehen lassen, welche federnd gegen den generellen Verlauf der Stege 7 beweglich sind. Dies ermöglicht es, zwischen die Federzungen 6 und die umgebenden Verbindungsstege 7 eine Membran einzuschieben, die dort klemmend festgehalten wird. Es versteht sich ferner, dass die Verbindungsstege zwischen den einzelnen Ringsegmenten durchaus unterschiedlich ausgebildet sein können, beispielsweise gestreckt zwischen einem endständigen Ringsegment und dem benachbarten Ringsegment und gekrümmt zwischen mittelständigen Ringsegmenten.

Die Ausführungsform gemäß Fig. 1 zeigt ferner frei endende Stege 9, die von den Stegschlingen 5 ausgehen und parallel zu den Verbindungsstegen 7 mit den Federzungen 6 ausgerichtet sind. Diese frei endenden Stege 9 sind ebenfalls gegen den Verlauf der Verbindungsstege 7 und der Ringsegmente 3 in die gleiche Richtung beweglich, wie die Federzungen 6. Ein Membran kann alternierend zwischen die Federzungen 6 und die frei endenden Stege 9 eingeklemmt werden, dergestalt, dass die Membran unterhalb der Federzungen 6 und oberhalb der frei endenden Stege 9 zu liegen kommt. Die Stege 9 verbessern den Halt der Membran am Stent.

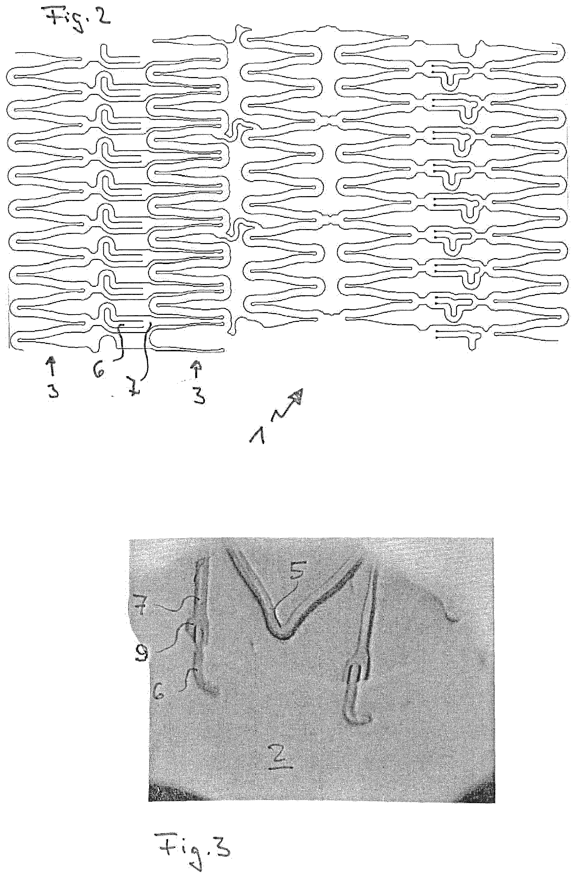

Fig. 2 zeigt eine bevorzugte Variante eines erfindungsgemäßen Stent-Grafts. In dieser Variante sind die Federzungen 6, die in den Verbindungsstegen 7 zwischen den randständigen Segmenten 3 angeordnet sind, abgewinkelt. Die Federzungen 6 haben damit ein hakenförmiges Design, sind aber nach wie vor formschlüssig in den Verbindungssteg 7 eingepasst. Im Bereich der Abwinklung weist der Verbindungssteg 7 eine halbkreisförmige Krümmung auf, die den abgewinkelten Teil der Federzunge 6 einschließt. Die Federzunge 6 ist nach wie vor gegen den Verlauf des Verbindungsstegs federnd beweglich. Federzunge und angrenzende Stegelemente haben eine im Wesentlichen gleiche Breite.

Dieser besondere Verlauf der Federzunge 6 bringt eine größere Kontaktfläche zur eingeklemmten Membran mit sich und dient damit der rutschfesten Absicherung der Membran.

Fig. 3 zeigt den Stent gemäß Fig. 2 mit eingespannter Membran. Zu erkennen ist die Stegschlinge 5 des Ringsegments, von dem der Verbindungssteg ausgeht, die Gabel 10 des Verbindungsstegs 7 mit dem Ansatz des Federelements 6 und das abgekröpfte Ende des Federelements 6. Die Membran 2 ist zwischen die Verbindungsstege 7 im gegabelten Bereich und der Federzunge 6 eingespannt, dergestalt, dass die Federzunge 6 im Wesentlichen auf die Membran zu liegen kommt und diese gegen den darunter verlaufenden Verbindungssteg 7 festhält.

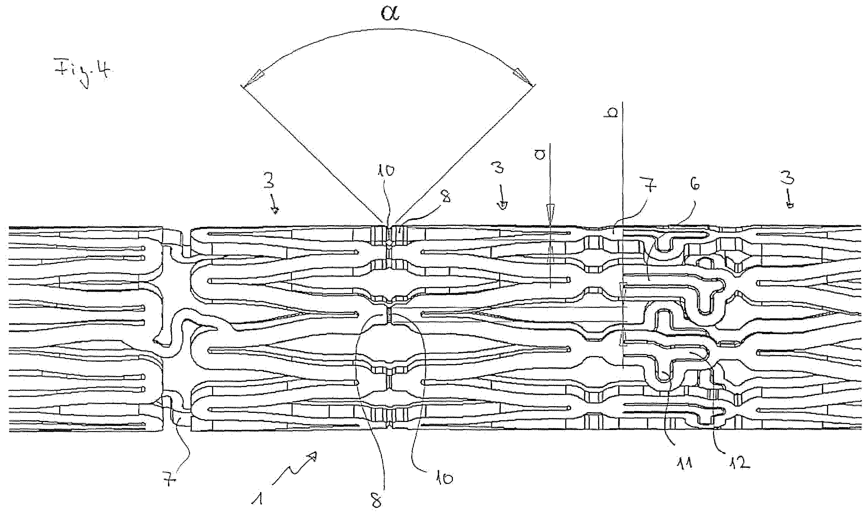

Fig. 4 zeigt einen erfindungsgemäß verwendbaren Stent 1 mit Einkerbungen 10. Die Einkerbungen 10 befinden sich außen auf Verbindungsstegen 8 zwischen zwei Ringsegmenten 3, die keine Federzungen 6 aufweisen. Die Einkerbungen 10 weisen gerade Schenkel mit einem Öffnungswinkel α von 90° auf und haben eine Tiefe von etwa 1/5 der Stegdicke der Verbindungsstege 8. Die Stegbreite b der Verbindungsstege 8 ist von etwa 1/4 im Bereich der Einkerbung 10 reduziert, a gibt die reduzierte Dicke der Verbindungsstege 8 im Bereich der Einkerbungen 10 an.

Fig. 4 zeigt zudem eine Variante der Federzungen 6 in den Verbindungsstegen 7 zwischen einem Randsegment 3 und einem angrenzenden Ringsegment 3. Die Federzungen 6 haben hier ein abgewinkeltes Ende 11 (90°) mit einer im Bereich der Abwinklung angeordneten Nase 12, die zum Stentende weist.

Er erfindungsgemäße Stent-Graft wird in üblicher Weise auf einen Ballonkatheter gecrimpt und mit üblicher Technik am Einsatzort expandiert. Dabei legt sich der Stent 1 mit der Membran 2 an die Gefäßwand an. Die Membran 2 befindet sich zwischen Stent 1 und Gefäßwand.

## Patentansprüche

1. Stent-Graft aus einem Stent (1) mit einer Mehrzahl von nebeneinander angeordneten und durch Verbindungsstege (7) miteinander verbundenen Ringsegmenten (3) mit mäandrierendem Verlauf und wenigstens einer Membran (2), **dadurch gekennzeichnet, dass** eine Mehrzahl von Verbindungsstegen (7) zwischen benachbarten Ringsegmenten (3) Federzungen (6) aufweisen, die formschlüssig in den Verbindungsstegen (7) angeordnet sind und gegen die Verbindungsstege (7) federnd beweglich sind, wobei die Membran (2) zwischen Federzungen (6) und Verbindungsstegen (7) eingeklemmt ist.

2. Stent-Graft nach Anspruch 1, **dadurch gekennzeichnet, dass** wenigstens ein endständiges Ringsegment (3) über Verbindungsstege (7) mit Federzungen (6) mit einem benachbarten Ringsegment (3) verbunden ist.

3. Stent-Graft nach Anspruch 1 oder 2, **dadurch gekennzeichnet, dass** die Federzungen (6) stentauswärts weisen.

4. Stent-Graft nach Anspruch 3, **dadurch gekennzeichnet, dass** der Stent (1) eine Membran (2) in Form eines Schlauches aufweist.

5. Stent-Graft nach Anspruch 4, **dadurch gekennzeichnet, dass** die Membran (2) den Stent (1) außen bedeckt.

6. Stent-Graft nach Anspruch 5, **dadurch gekennzeichnet, dass** der Randbereich der in den Federzungen (6) eingeklemmten Membran (2) nach innen umgeschlagen ist.

7. Stent-Graft nach einem der vorstehenden Ansprüche, **dadurch gekennzeichnet, dass** die Membran (2) durch zusätzliches Verkleben mit Klebeband in den Federzungen (6) gesichert ist.

8. Stent-Graft nach einem der Ansprüche 1 bis 6, **gekennzeichnet durch** mehrere abschnittsweise angeordnete Membranen (2), die jeweils an Federzungen (6) gesichert sind.

9. Stent-Graft nach einem der vorstehenden Ansprüche, **dadurch gekennzeichnet, dass** die Membran (2) aus ePTFE-Schlauch besteht.

10. Stent-Graft nach einem der vorstehenden Ansprüche, **dadurch gekennzeichnet, dass** der Stent (1) ein Ballon-expandierbarer Stent ist.

11. Stent-Graft nach Anspruch 10, aufgekrimpt auf den Ballon eines Ballonkatheters.

12. Stent-Graft nach einem der Ansprüche 1 bis 9 mit einem selbst-expandierenden Stent aus einer Formgedächtnislegierung, insbesondere Nitinol.

13. Stent-Graft nach einem der vorstehenden Ansprüche, **dadurch gekennzeichnet, dass** die Federzungen (6) an ihrem freien Ende in der Ebene des Stents (1) abgewinkelt sind oder in ihrem Verlauf eine Abzweigung (11) aufweisen.

14. Stent-Graft nach einem der vorstehenden Ansprüche, **dadurch gekennzeichnet, dass** wenigstens eines der durch die Verbindungsstege (7) mit den Federzungen (6) verbundenen Ringsegmente (3) an seinen Umkehrpunkten in dem Zwischenraum zwischen zwei Verbindungsstegen (7) ragende frei endende Stege (9) aufweist, die der zusätzlichen Fixierung der Membran 82) dienen.

15. Stent-Graft nach einem der vorstehenden Ansprüche, **gekennzeichnet durch** Einkerbungen (10) in Verbindungsstege (6) zwischen zwei Ringsegmenten (3).

16. Stent-Graft nach Anspruch 15, **dadurch gekennzeichnet, dass** die Einkerbungen (10) in alle Verbindungsstege (8) zwischen zwei Ringsegmenten (3) eingebracht sind und eine Tiefe von nicht mehr als 1/3 der Stegdicke aufweisen.

## Claims

1. Stent graft comprising a stent (1) and at least one membrane (2), the stent having a plurality of ring segments (3) arranged side by side, the plurality of ring segments having a meandering pattern and being connected with each other by connecting webs (7), **characterized in that**
a plurality of connecting webs (7) between adjacent ring segments (3) are provided with flexible tongues (6), which are arranged in a form-closed manner in the connecting webs (7) and which are resiliently movable against the connecting webs (7), wherein the membrane (2) is clamped between flexible tongues (6) and connecting webs (7).

2. Stent graft according to claim 1, **characterized in that** at least one peripheral ring segment (3) is connected to an adjacent ring segment (3) via connecting webs (7) with flexible tongues (6).

3. Stent graft according to claim 1 or 2, **characterized in that** the flexible tongues (6) point out of the stent.

4. Stent graft according to claim 3, **characterized in that** the stent (1) is provided with a membrane (2) in the form of a tube.

5. Stent graft according to claim 4, **characterized in that** the membrane (2) covers the outside of the stent (1).

6. Stent graft according to claim 5, **characterized in that** the edge region of the membrane (2) clamped in place via the flexible tongues (6) is folded inwardly.

7. Stent graft according to any one of the preceding claims, **characterized in that** the membrane (2) is secured in the flexible tongues (6) by additional bonding with adhesive tape.

8. Stent graft according to any one of claims 1 to 6, **characterized by** a plurality of membranes (2) arranged in sections, each of which is secured to flexible tongues (6).

9. Stent graft according to any one of the preceding claims, **characterized in that** the membrane (2) consists of ePTFE tube.

10. Stent graft according to any one of the preceding claims, **characterized in that** the stent (1) is a balloon-expandable stent.

11. Stent graft according to claim 10, crimped onto the balloon of a balloon catheter.

12. Stent graft according to any one of claims 1 to 9 with a self-expanding stent made of a shape-memory alloy, in particular nitinol.

13. Stent graft according to any one of the preceding claims, **characterized in that** the flexible tongues (6) are of angled configuration at their free end in the plane of the stent (1) or have a branch (11) in their configuration.

14. Stent graft according to any one of the preceding claims, **characterized in that** at least one of the ring segments (3) connected with each other by the connecting webs (7) with flexible tongues (6) has freely ending webs (9) at their reversal points projecting into the gap between two connecting webs (7), said free webs serving to additionally secure the membrane (2).

15. Stent graft according to any one of the preceding claims, **characterized by** notches (10) provided in connecting webs (7) between two ring segments (3).

16. Stent graft according to claim 15, **characterized in that** all connecting webs (8) between two ring segments (3) comprise notches (10), the notches have a depth of not more than 1/3 of the web thickness.

## Revendications

1. Endoprothèse couverte composée d'une endoprothèse (1) et d'une multitude de segments annulaires (3) à profil sinueux disposés côte à côte et reliés les uns aux autres par des entretoises de liaison (7) et d'au moins une membrane (2), **caractérisée en ce qu'**une multitude d'entretoises de liaison (7) présentent entre des segments annulaires (3) adjacents des languettes élastiques (6), qui sont disposées par complémentarité de forme dans les entretoises de liaison (7) et qui sont mobiles sur ressorts contre les entretoises de liaison (7), dans laquelle la membrane (2) est coincée entre des languettes élastiques (6) et des entretoises de liaison (7).

2. Endoprothèse couverte selon la revendication 1, **caractérisée en ce qu'**au moins un segment annulaire (3) en position terminale est relié à un segment annulaire (3) adjacent par des languettes élastiques (6) par l'intermédiaire d'entretoises de liaison (7).

3. Endoprothèse couverte selon la revendication 1 ou 2, **caractérisée en ce que** les languettes élastiques (6) pointent vers l'extérieur de l'endoprothèse.

4. Endoprothèse couverte selon la revendication 3, **caractérisée en ce que** l'endoprothèse (1) présente une membrane (2) sous la forme d'un tuyau flexible.

5. Endoprothèse couverte selon la revendication 4, **caractérisée en ce que** la membrane (2) recouvre côté extérieur l'endoprothèse (1).

6. Endoprothèse couverte selon la revendication 5, **caractérisée en ce que** la zone de bord de la membrane (2) coincée dans les languettes élastiques (6) est retournée vers l'intérieur.

7. Endoprothèse couverte selon l'une quelconque des revendications précédentes, **caractérisée en ce que** la membrane (2) est bloquée dans les languettes élastiques (6) avec une bande adhésive par collage supplémentaire.

8. Endoprothèse couverte selon l'une quelconque des revendications 1 à 6, **caractérisée par** plusieurs membranes (2) disposées par endroits, qui sont bloquées respectivement sur des languettes élastiques (6).

9. Endoprothèse couverte selon l'une quelconque des revendications précédentes, **caractérisée en ce que** la membrane (2) est constituée d'un tuyau flexible en ePTFE.

10. Endoprothèse couverte selon l'une quelconque des revendications précédentes, **caractérisée en ce que** l'endoprothèse (1) est une endoprothèse expansible par ballonnet.

11. Endoprothèse couverte selon la revendication 10, sertie sur le ballonnet d'un cathéter à ballonnet.

12. Endoprothèse couverte selon l'une quelconque des revendications 1 à 9 avec une endoprothèse auto-expansible composée d'un alliage à mémoire de forme, en particulier de nitinol.

13. Endoprothèse couverte selon l'une quelconque des revendications précédentes, **caractérisée en ce que** les languettes élastiques (6) sont coudées sur leur extrémité libre dans le plan de l'endoprothèse (1) ou présentent une ramification (11) dans leur profil.

14. Endoprothèse couverte selon l'une quelconque des revendications précédentes, **caractérisée en ce qu'**au moins un des segments annulaires (3) reliés aux languettes élastiques (6) par les entretoises de liaison (7) présente sur ses points d'inversion dans l'espace intermédiaire des entretoises (9) se terminant librement dépassant entre deux entretoises de liaison (7), qui servent à la fixation supplémentaire de la membrane (82).

15. Endoprothèse couverte selon l'une quelconque des revendications précédentes, **caractérisée par** des encoches (10) dans des entretoises de liaison (6) entre deux segments annulaires (3).

16. Endoprothèse couverte selon la revendication 15, **caractérisée en ce que** les encoches (10) sont pratiquées dans la totalité des entretoises de liaison (8) entre deux segments annulaires (3) et présentent une épaisseur ne dépassant pas 1/3 de l'épaisseur d'entretoise.
